# EUROPEAN PATENT SPECIFICATION

(11) **EP 4 169 546 B1**
(45) Date of publication and mention of the grant of the patent: **17.12.2025**
(21) Application number: 21203989.5
(22) Date of filing: 21.10.2021
(51) Int. Cl.: A61M 5/14

(54) **MULTIPURPOSE UTILITY HOLDER FOR IV POLE**
MEHRZWECKHALTER FÜR EINEN INFUSIONSSTÄNDER
SUPPORT D'UTILITÉ MULTIFONCTION POUR PÔLE IV

(43) Date of publication of application: 26.04.2023
(73) Proprietor: Millar, Allen Currie, Rogers, AR 72758 (US)
(72) Inventor: Millar, Allen Currie, Rogers, AR 72758 (US)
(74) Representative: D Young & Co LLP

(56) References cited:
- CN-A- 111 420 161
- US-A- 5 588 166
- US-A1- 2014 209 550
- US-A1- 2015 157 522

## Description

### Field of the Invention

The present invention relates to devices for holding articles, such as (i) additional IV bags, bottles or other containers for intravenous fluid therapies, (ii) pacemakers, (iii) ether screens, (iv) transesophageal echo probes, and/or (v) other articles, on IV poles.

### Background of the Invention

IV poles are used in operating rooms, cardiac catheterization labs, endoscopic labs, intensive care units, and in other medical treatment areas and facilities for hanging fluid bags, bottles, and other containers for intravenous therapies. In addition, various makeshift devices have also been used on IV poles for hanging pacemakers, ether screens (which shield the anesthesia personnel from the surgical field), transesophageal echo probes, and other articles.

Unfortunately, the makeshift devices heretofore used in the art for holding other articles on IV poles have been deficient in many important respects. Typically, the ether screen has been held by a simple clip and the pacemaker has been hung by a homemade hook formed by bending a stylet (i.e., a soft metal rod). In addition to being unstable, the homemade hook holds the pacemaker up high at a point above the ether screen so that the pacemaker is exposed to blood and other contaminants from the surgical field. Moreover, the stylet, which can only be used once, must be thrown away after just a single use.

Consequently, a need exists for an improved device for holding an ether screen, a pacemaker, transesophageal echo probes, and other articles on an IV pole. The device will preferably hold the pacemaker and other articles in a more secure manner and will preferably position the pacemaker behind, rather than above, the ether screen so that the pacemaker is shielded from the surgical field. The improved device will also preferably be cleanable and reusable. In addition, the improved device will preferably allow additional IV fluid bags, bottles, or other containers to be hung from the IV pole.

US 2015/157522 A1 describes a pole system for supporting medical equipment having a base with legs, a mast engaged to the base, and a lifting mechanism constructed to position the legs in an extended configuration and a retracted configuration in which the legs are raised relative to a support surface proximate to the mast.

US 5,588,166 A describes a medical attachment device that is hung upon and rigidly attached to an upright and horizontally disposed part of a patient transport vehicle and that also grasps an upright pole of a wheeled patient care apparatus for maintaining the vehicle and the apparatus in fixed spatial relationship while both are being moved by a single medical attendant.

### Summary of the Invention

The present invention provides a multipurpose utility holder for an IV pole which alleviates the problems discussed above. The inventive multipurpose holder is cleanable and reusable and can be used to securely hold multiple different types of articles on an IV pole in a much safer and more stable manner. Examples of such articles include but are not limited to (i) additional IV bags, bottles, or other containers for intravenous fluid therapies, (ii) pacemakers, (iii) ether screens, and (iv) transesophageal echo probes. The multipurpose utility holder will also position articles such as pacemakers behind, rather than above, the ether screen so that they are shielded from the surgical field.

According to the invention, there is provided a utility holder for an IV pole according to claim 1.

Particular and preferred aspects of the invention are set out in the appended claims.

Further aspects, features and advantages of the present invention will be apparent to those in the art upon examining the accompanying drawings and upon reading the following Detailed Description of the Preferred Embodiments.

### Brief Description of the Drawings

Fig. 1 is a perspective view of an embodiment **2** of the inventive multipurpose utility holder.
Fig. 2 is another perspective view of the inventive utility holder **2.**
Fig. 3 is an elevational side view of the inventive utility holder **2.**
Fig. 4 is another elevational view of the inventive utility holder **2.**
Fig. 5 is a perspective view of an alternative embodiment **80** of the inventive multipurpose utility holder.

### Detailed Description of the Preferred Embodiments

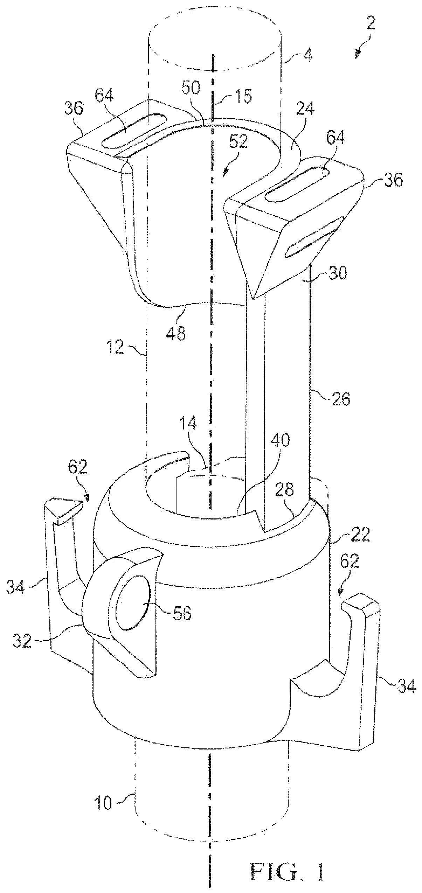

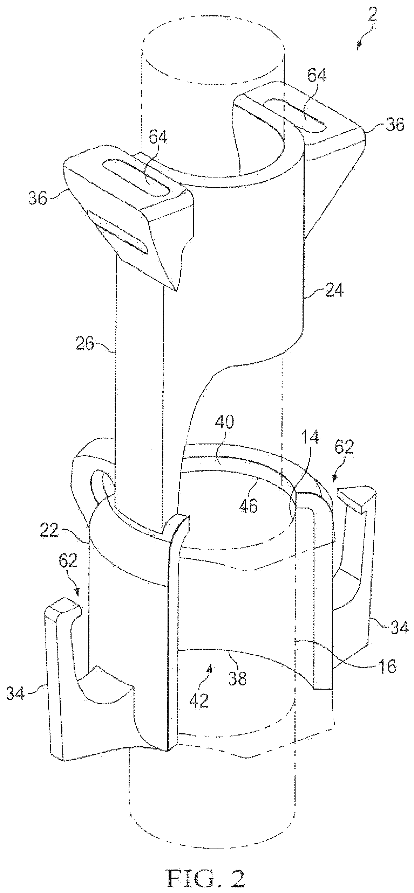

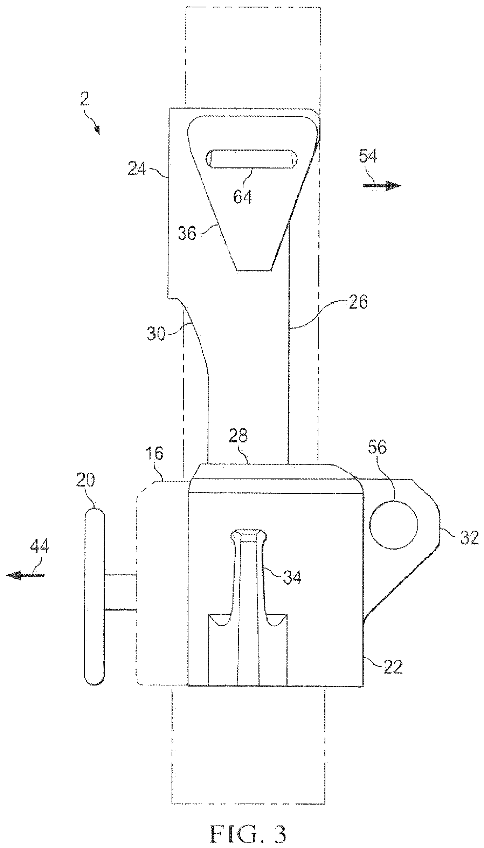

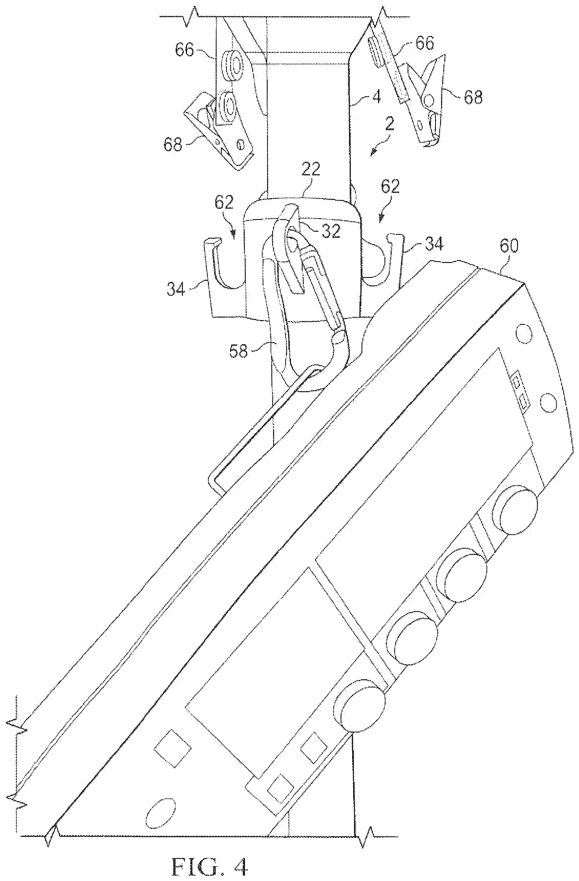

An embodiment **2** of the inventive multipurpose utility holder is shown in Figs. 1-4. As illustrated in Figs. 1-4, the multipurpose holder **2** is removably secured on an IV pole apparatus **4.** The IV pole apparatus **4** can be any type of IV pole apparatus which is known in the art. The IV pole apparatus **4** will typically comprise: a weighted base with wheels (not shown); a lower pole segment **10** which extends upwardly from the weighted base; an upper pole segment **12** which is slideably received in the lower pole segment **10** so that the upper pole segment telescopingly extends upwardly through the upper end **14** of the lower pole segment **10;** a locking collar **16** on the upper end **14** of the lower pole segment **10** for releasably locking the upper pole segment **12** at a desired height selected by the user; and a set of hooks at the top of the upper pole segment **12** for hanging bags, bottles, or other containers containing fluids used for intravenous medical therapies. The locking collar **16** can be (a) a knob-type collar which comprises a locking bolt which extends radially through the collar **16** and has a hand knob **20** on the outer end thereof for tightening and releasing the locking collar **16,** (b) a twist-type collar which is threadedly received on the upper end **14** of the lower pole segment **10,** or (c) any other type of locking collar device used in the art.

The multipurpose utility holder **2** preferably comprises: a longitudinal axis **15;** an open lower collar **22** in which the locking collar **16** of the IV pole **4** is removably received; an open upper collar **24** through which the upper pole segment **12** of the IV pole **4** is removably received; a longitudinally extending connecting arm **26** having a lower end **28** which is connected to the open lower collar **22** and an upper end **30** which is connected to the open upper collar **24;** one or more attachment structures **32, 34** which project from the open lower collar **22;** and one or more attachment structures **36** which project from the open upper collar **24.**

Although other fabrication techniques can alternatively be used, the multipurpose utility holder **2** is preferably a unitary molded structure which is formed of a non-porous plastic material. The plastic material will preferably be both (a) sufficiently flexible for placing the inventive utility holder **2** on the IV pole apparatus **4** and (b) sufficiently resilient for tightly gripping the IV pole **4** when the utility holder **2** is in use for holding multiple articles.

The open lower collar **22** of the inventive utility holder **2** has an open lower end **38** and open upper end **40.** The open lower collar **22** also has a lateral cross-sectional C-shape such that the open lower collar **22** has a side gap opening **42** which faces in a first radial direction **44.** The open lower collar **22** preferably extends from about 190° to about 320° (i.e., within ± 5°) around both (i) the longitudinal axis **15** of the inventive holder **2** and (ii) the IV pole **4.** The open lower collar **22** more preferably extends at least 200° and most preferably extends from about 220° to about 280° around the longitudinal axis **15** and the IV pole **4.**

The material used for forming the inventive utility holder **2** will preferably allow the side gap opening **42** of the open lower collar **22** to flex to a degree which is sufficient for receiving at least the upper pole segment **12** of the IV pole **4** for placing the open lower collar **22** on the locking collar **16.**

The open lower collar **22** preferably also includes a retaining shoulder **46,** located at the upper longitudinal end **40** of the lower collar **22,** which rests on the upper end of the locking collar **16** of the IV pole **4** when the locking collar **16** is received in open lower collar **22.** The retaining shoulder **46** extends radially inward toward the longitudinal axis **15** and is preferably an inwardly curved shoulder. As with the remainder of the open lower collar **22,** the retaining shoulder **46** preferably extends from about 190° to about 320°, more preferably at least 200° and more preferably from about 220° to about 280°, around the longitudinal axis **15.**

The open upper collar **24** of the inventive utility holder **2** has an open lower end **48** and open upper end **50.** The open upper collar **24** also has a lateral cross-sectional C-shape such that the open upper collar **24** has a side gap opening **52** which faces in a second radial direction **54.** The open upper collar **24** preferably extends from about 190° to about 320° (i.e., within ± 5°) around both (i) the longitudinal axis **15** of the inventive holder **2** and (ii) the IV pole **4.** The open upper collar **24** more preferably extends at least 200° and most preferably extends from about 220° to about 280° around the longitudinal axis **15** and the IV pole **4.**

The material used for forming the inventive utility holder **2** will preferably allow the side gap opening **52** of the open upper collar **24** to flex to a degree which is sufficient for receiving the upper pole segment **12** of the IV pole **4** to place the open upper collar **24** on the upper pole segment **12.**

As noted above, the side gap opening **42** of the open lower collar **22** faces in a first radial direction **44** and the side gap opening **52** of the open upper collar **24** faces in a second radial direction **54.** The second radial direction **54** is preferably different from the first radial direction **44** and is more preferably about 180° opposite the first radial direction **44.** In addition, the connecting arm **26** of the inventive multipurpose utility holder **2** preferably extends longitudinally along only one side of the utility holder **2** and does not block or interfere with either the side gap opening **42** of the open lower collar **22** or the side gap opening **52** of the open upper collar **24.** This configuration allows the inventive utility holder **2** to be snapped onto the IV pole apparatus **4** without disassembling the IV pole **4.**

The one or more attachment structures **32, 34** which project outwardly from the open lower collar **22** of the multipurpose utility holder **2** and the one or more attachment structures **36** which project outwardly from the open upper collar **24** can be any type of structures suitable for holding instruments, screens (e.g., drapes), tubes, probes, additional IV bags, and/or other articles on the IV pole **4** for use in a surgical operating room, a cardiac catheterization lab, an endoscopic lab, an intensive care unit, or other medical treatment area or facility.

At least one of the attachment structures provided on the open lower collar **22** is preferably an ear-shaped attachment lug **32** having an aperture **56** extending therethrough for, e.g., receiving and holding a carabiner **58** for attaching a pacemaker **60** or other instrument to the open lower collar **22.** Alternatively, or in addition, at least one, preferably two, of the attachment structures on the open lower collar **22** is/are hook structure(s) **34.** Each hook **34** preferably has an upper opening **62** and will receive and hold, e.g., an IV bag or a transesophageal echo probe.

At least one, preferably two, of the attachment structures provided on the open upper collar **24** is/are attachment lug(s) **36,** each of which has a slot **64** extending therethrough, e.g., for receiving and holding a band **66** of a band clip **68** for attaching an ether screen to the open upper collar **24.**

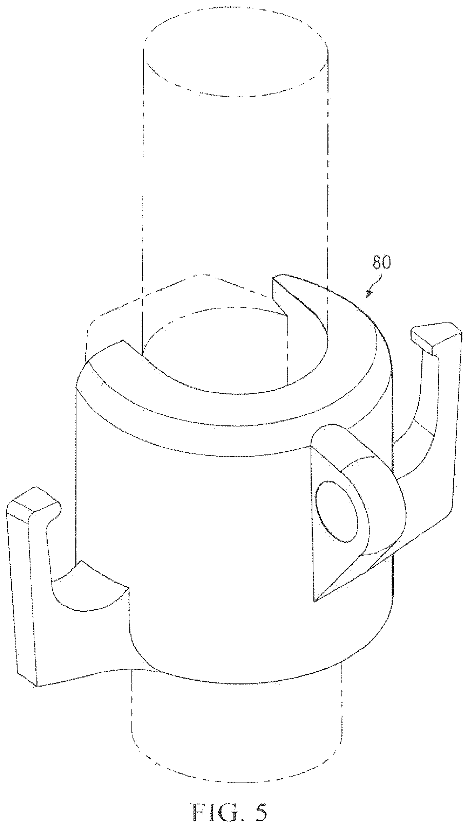

An alternative embodiment **80** of the inventive multipurpose utility holder is illustrated in Fig. 5. The inventive utility holder **80** is identical to the inventive utility holder **2** except that the utility holder **80** does not include the open upper collar **24** of the inventive holder **2** and does not include the connecting arm **26** of the holder **2** which extends between the open lower collar **22** and the open upper collar **24.**

Thus, the present invention is well adapted to carry out the objects and attain the ends and advantages mentioned above as well as those inherent therein. While presently preferred embodiments have been described for purposes of this disclosure, numerous changes and modifications will be apparent to those in the art. Such changes and modifications are encompassed within this invention, provided they fall within the scope of the appended set of claims.

## Claims

1. A utility holder (2) for an IV pole (4), the utility holder comprising:
a longitudinal axis;
an open lower collar (22) which extends from about 190° to about 320° around the longitudinal axis, the open lower collar having a lateral cross-sectional C-shape;
an open upper collar (24) which extends from about 190° to about 320° around the longitudinal axis, the open upper collar being positioned longitudinally above the open lower collar and the open upper collar having a lateral cross-sectional C-shape;
a connecting arm (26) which extends longitudinally from the open lower collar to the open upper collar;
one or more attachment structures (32, 34) which project outwardly from the open lower collar;
the open lower collar having a flexible side gap opening (42);
the open upper collar having a flexible side gap opening (52);
the flexible side gap opening of the open lower collar facing a first radial direction with respect to the longitudinal axis;
**characterised in that**
the flexible side gap opening of the open upper collar faces a second radial direction with respect to the longitudinal axis which is about 180° opposite the first radial direction.

2. The utility holder of claim 1 further comprising the open lower collar having a retaining shoulder (46) at an upper longitudinal end of the open lower collar which extends radially inward toward the longitudinal axis.

3. The utility holder of claim 2 further comprising the retaining shoulder being an inwardly curved shoulder.

4. The utility holder of any preceding claim further comprising the one or more attachment structures comprising an attachment lug (32) projecting outwardly from the open lower collar, the attachment lug having an aperture (56) extending therethrough.

5. The utility holder of claim 4 further comprising a carabiner (58) attached to the open lower collar through the aperture of the attachment lug.

6. The utility holder of any preceding claim further comprising the one or more attachment structures comprising one or more hooks (34) which extend outwardly from the open lower collar.

7. The utility holder of any preceding claim further comprising:
one or more attachment lugs (36) which project outwardly from the open upper collar, each of the one or more attachment lugs having a slot (64) extending therethrough; and
the slot of at least one of the one or more attachment lugs having a band (66) of a band clip (68) extending therethrough.

8. The utility holder of any of claims 2 to 7 further comprising:
an IV pole apparatus (4) comprising a lower pole segment (10) , an upper pole segment (12) which telescopingly extends through an upper end (14) of the lower pole segment, and a locking collar (16) on the upper end of the lower pole segment for locking the upper pole segment at a selected height and
the locking collar of the IV pole apparatus being received in the open lower collar such that the retaining shoulder of the open lower collar rests on the locking collar.

9. The utility holder of claim 8 comprising the upper pole segment of the IV pole apparatus being received through the open upper collar.

10. The utility holder of any preceding claim in which the connecting arm extends longitudinally along only one side of the utility holder, the connecting arm not blocking the flexible side gap opening of the open lower collar, and the connecting arm not blocking the flexible side gap opening of the open upper collar.

## Patentansprüche

1. Hilfsmittelhalter (2) für einen Infusionsständer (4), wobei der Hilfsmittelhalter Folgendes umfasst:
eine Längsachse;
eine offene untere Manschette (22), die sich von etwa 190° bis etwa 320° um die Längsachse erstreckt, wobei die offene untere Manschette einen seitlichen C-förmigen Querschnitt aufweist;
eine offene obere Manschette (24), die sich von etwa 190° bis etwa 320° um die Längsachse erstreckt, wobei die offene obere Manschette in Längsrichtung oberhalb der offenen unteren Manschette positioniert ist und die offene obere Manschette einen seitlichen C-förmigen Querschnitt aufweist;
einen Verbindungsarm (26), der sich in Längsrichtung von der offenen unteren Manschette zur offenen oberen Manschette erstreckt;
eine oder mehrere Befestigungsstrukturen (32, 34), die von der offenen unteren Manschette nach außen vorstehen;
wobei die offene untere Manschette eine flexible Seitenspaltöffnung (42) aufweist;
wobei die offene obere Manschette eine flexible Seitenspaltöffnung (52) aufweist;
wobei die flexible Seitenspaltöffnung der offenen unteren Manschette einer ersten radialen Richtung in Bezug auf die Längsachse zugewandt ist;
**dadurch gekennzeichnet, dass** die flexible Seitenspaltöffnung der offenen oberen Manschette einer zweiten radialen Richtung in Bezug auf die Längsachse zugewandt ist, die etwa 180° entgegengesetzt zur ersten radialen Richtung verläuft.

2. Hilfsmittelhalter nach Anspruch 1, ferner umfassend die offene untere Manschette mit einer Halteschulter (46) an einem oberen Längsende der offenen unteren Manschette, die sich radial nach innen in Richtung der Längsachse erstreckt.

3. Hilfsmittelhalter nach Anspruch 2, ferner umfassend, dass die Halteschulter eine nach innen gekrümmte Schulter ist.

4. Hilfsmittelhalter nach einem der vorhergehenden Ansprüche, ferner umfassend die eine oder die mehreren Befestigungsstrukturen, die eine Befestigungsnase (32) umfassen, die von der offenen unteren Manschette nach außen vorsteht, wobei die Befestigungsnase eine Öffnung (56) aufweist, die sich durch sie hindurch erstreckt.

5. Hilfsmittelhalter nach Anspruch 4, ferner umfassend einen Karabiner (58), der durch die Öffnung der Befestigungsnase an der offenen unteren Manschette befestigt ist.

6. Hilfsmittelhalter nach einem der vorhergehenden Ansprüche, ferner umfassend die eine oder die mehreren Befestigungsstrukturen, die einen oder mehrere Haken (34) umfassen, die sich von der offenen unteren Manschette nach außen erstrecken.

7. Hilfsmittelhalter nach einem der vorhergehenden Ansprüche, ferner umfassend:
eine oder mehrere Befestigungsnasen (36), die von der offenen oberen Manschette nach außen vorstehen, wobei jede der einen oder mehreren Befestigungsnasen einen Schlitz (64) aufweist, der sich durch sie hindurch erstreckt; und
wobei der Schlitz von mindestens einer der einen oder mehreren Befestigungsnasen ein Band (66) einer Bandklammer (68) aufweist, das sich durch sie hindurch erstreckt.

8. Hilfsmittelhalter nach einem der Ansprüche 2 bis 7, ferner umfassend:
eine Infusionsständervorrichtung (4), umfassend ein unteres Ständersegment (10), ein oberes Ständersegment (12), das sich teleskopartig durch ein oberes Ende (14) des unteren Ständersegments erstreckt, und eine Verriegelungsmanschette (16) am oberen Ende des unteren Ständersegments zum Verriegeln des oberen Ständersegments in einer ausgewählten Höhe und
wobei die Verriegelungsmanschette der Infusionsständervorrichtung derart in der offenen unteren Manschette aufgenommen ist, dass die Halteschulter der offenen unteren Manschette auf der Verriegelungsmanschette aufliegt.

9. Hilfsmittelhalter nach Anspruch 8, wobei das obere Ständersegment der Infusionsständervorrichtung durch die offene obere Manschette aufgenommen wird.

10. Hilfsmittelhalter nach einem der vorhergehenden Ansprüche, wobei sich der Verbindungsarm in Längsrichtung nur entlang einer Seite des Hilfsmittelhalters erstreckt, wobei der Verbindungsarm die flexible Seitenspaltöffnung der offenen unteren Manschette nicht blockiert und der Verbindungsarm die flexible Seitenspaltöffnung der offenen oberen Manschette nicht blockiert.

## Revendications

1. Support pour accessoires (2) pour une potence à perfusion (4), le support pour accessoires comprenant :
un axe longitudinal ;
une bague inférieure ouverte (22) qui s'étend d'environ 190° à environ 320° autour de l'axe longitudinal, la bague inférieure ouverte présentant une section latérale en C ;
une bague supérieure ouverte (24) qui s'étend d'environ 190° à environ 320° autour de l'axe longitudinal, la bague supérieure ouverte étant positionnée longitudinalement au-dessus de la bague inférieure ouverte et la bague supérieure ouverte présentant une section latérale en C ;
un bras de liaison (26) qui s'étend longitudinalement de la bague inférieure ouverte à la bague supérieure ouverte ;
une ou plusieurs structures de fixation (32, 34) qui font saillie vers l'extérieur depuis la bague inférieure ouverte ;
la bague inférieure ouverte comportant une ouverture d'espacement latérale flexible (42) ;
la bague supérieure ouverte comportant une ouverture d'espacement latérale flexible (52) ;
l'ouverture d'espacement latérale flexible de la bague inférieure ouverte étant orientée dans une première direction radiale par rapport à l'axe longitudinal ;
**caractérisé en ce que** l'ouverture d'espacement latérale flexible de la bague supérieure ouverte est orientée dans une seconde direction radiale par rapport à l'axe longitudinal qui est opposée à environ 180° à la première direction radiale.

2. Support pour accessoires selon la revendication 1, dans lequel en outre la bague inférieure ouverte comporte un épaulement de retenue (46) à une extrémité longitudinale supérieure de la bague inférieure ouverte qui s'étend radialement vers l'intérieur en direction de l'axe longitudinal.

3. Support pour accessoires selon la revendication 2, dans lequel en outre l'épaulement de retenue est un épaulement incurvé vers l'intérieur.

4. Support pour accessoires selon l'une quelconque des revendications précédentes, dans lequel en outre la ou les structures de fixation comprennent une patte de fixation (32) faisant saillie vers l'extérieur à partir de la bague inférieure ouverte, la patte de fixation ayant une ouverture (56) s'étendant à travers elle.

5. Support pour accessoires selon la revendication 4, comprenant en outre un mousqueton (58) fixé à la bague inférieure ouverte à travers l'ouverture de la patte de fixation.

6. Support pour accessoires selon l'une quelconque des revendications précédentes, dans lequel en outre la ou les structures de fixation comprennent un ou plusieurs crochets (34) qui s'étendent vers l'extérieur à partir de la bague inférieure ouverte.

7. Support pour accessoires selon l'une quelconque des revendications précédentes, comprenant en outre :
une ou plusieurs pattes de fixation (36) qui font saillie vers l'extérieur à partir de la bague supérieure ouverte, la ou chacune de ces pattes de fixation présentant une fente (64) qui la traverse ; et
la fente de la ou d'au moins une des pattes de fixation comportant une lanière (66) d'une pince à lanière (68) s'étendant à travers elle.

8. Support pour accessoires selon l'une quelconque des revendications 2 à 7, comprenant en outre :
un appareil de potence à perfusion (4) comprenant un segment de potence inférieur (10), un segment de potence supérieur (12) qui s'étend de manière télescopique à travers une extrémité supérieure (14) du segment de potence inférieur, et une bague de verrouillage (16) sur l'extrémité supérieure du segment de potence inférieur pour verrouiller le segment de potence supérieur à une hauteur sélectionnée et
la bague de verrouillage de l'appareil de potence à perfusion étant reçue dans la bague inférieure ouverte de sorte que l'épaulement de retenue de la bague inférieure ouverte soit en appui sur la bague de verrouillage.

9. Support pour accessoires selon la revendication 8, dans lequel le segment de potence supérieur de l'appareil de potence à perfusion est reçu à travers la bague supérieure ouverte.

10. Support pour accessoires selon l'une quelconque des revendications précédentes, dans lequel le bras de liaison s'étend longitudinalement le long d'un seul côté du support pour accessoires, le bras de liaison ne bloquant pas l'ouverture d'espacement latérale flexible de la bague inférieure ouverte, et le bras de liaison ne bloquant pas l'ouverture d'espacement latérale flexible de la bague supérieure ouverte.
